# EUROPÄISCHE PATENTANMELDUNG

(11) **EP 1 226 783 A2**
(43) Veröffentlichungstag der Anmeldung: **31.07.2002**
(21) Anmeldenummer: 02000805.8
(22) Anmeldetag: 14.01.2002
(51) Int. Cl.: A61B 5/00

(54) **Verfahren und medizinisches System zur Überwachung eines an Epilepsie leidenden Patienten**

(30) Priorität: 25.01.2001 DE 10103327
(71) Anmelder: SIEMENS AKTIENGESELLSCHAFT, 80333 München (DE)
(72) Erfinder: Schmidt, Volker, Dr., 91054 Erlangen (DE); Striebel, Werner, 90592 Schwarzenbruck (DE)

(57) **Zusammenfassung**

Die Erfindung betrifft ein Verfahren und ein medizinisches System zur Überwachung wenigstens eines an Epilepsie leidenden Patienten außerhalb einer medizinischen Einrichtung. Zur Überwachung des Patienten wird wenigstens ein das Epilepsieleiden betreffender Messwert des Patienten erfasst, mit einer Auswertevorrichtung (24) ausgewertet und in einer zentralen Datenbank (27) gespeichert. Durch einen Alarmgeber (24) wird ein Alarmsignal erster Art ausgelöst, wenn der ausgewertete Messwert als für den Gesundheitszustand des Patienten kritisch eingestuft wird, oder es wird ein Alarmsignal zweiter Art ausgelöst wird, wenn der Messwert des Patienten ausbleibt.

## Beschreibung

Die Erfindung betrifft ein Verfahren und ein medizinisches System zur Überwachung eines an Epilepsie leidenden Patienten außerhalb einer medizinischen Einrichtung.

Die effektive Behandlung von an Epilepsie leidenden Patienten durch einen Arzt erfordert patientenspezifische Informationen, welche beispielsweise den Typ eines epileptischen Anfalls, die Häufigkeit eines epileptischen Anfalls oder vom Patienten eingenommene Medikamente umfassen. Dies ist insbesondere bei medikamentös schwierig einzustellenden Patienten erforderlich, bei denen es immer wieder zu epileptischen Anfällen kommt.

Bisher ist es zur außerklinischen Überwachung eines an Epilepsie leitenden Patienten gängige Verfahrenspraxis, dass ein Patient zu Hause die für sein Krankheitsbild und seinen Krankheitsverlauf spezifischen Messwerte festhält und an einen behandelnden Arzt papiergebunden, beispielsweise per Fax, übermittelt, so dass der Arzt basierend auf den Messwerten eine Diagnose erstellen und eine geeignete Therapie des Patienten wählen kann.

Nachteilig an dieser Verfahrensweise ist, dass der Arzt die Mitarbeit des Patienten sowie die Zuverlässigkeit des Patienten bei der Ermittlung und der Übermittlung der Messwerte nur unter großem Verwaltungsaufwand überwachen kann. Daher besteht die Gefahr, dass insbesondere bei nachlässigen Patienten Verschlechterungen des Gesundheitszustandes oder akute Gesundheitsrisiken zu spät erkannt werden. Es ist zwar möglich, Messwerte durch ambulante Pflegedienste bei Patienten zu Hause erfassen zu lassen. Aufgrund von Medienbrüchen bei der Übertragung der Messwerte, von Problemen bei der Patientenidentifikation und aufgrund des hohen Personalaufwandes gelangen auch diese Messwerte jedoch häufig nicht oder erst verspätet zu einem behandelnden Arzt.

Der Erfindung liegt daher die Aufgabe zugrunde, ein Verfahren der eingangs genannten Art bzw. ein medizinisches System der eingangs genannten Art derart anzugeben, dass die Überwachung eines an Epilepsie leidenden Patienten außerhalb einer medizinischen Einrichtung verbessert wird.

Nach der Erfindung wird diese Aufgabe gelöst durch ein Verfahren gemäß Anspruch 1 und ein medizinisches System gemäß Anspruch 12. Zur Überwachung wenigstens eines an Epilepsie leidenden Patienten wird außerhalb einer medizinischen Einrichtung wenigstens ein, in der Regel mehrere das Epilepsieleiden betreffende Messwerte eines Patienten erfasst, an eine zentrale Datenbank übermittelt und dort gespeichert. Anschließend erfolgt eine Auswertung des oder der Messwerte, wobei ein Alarmsignal erster Art ausgelöst wird, wenn wenigstens einer der ausgewerteten Messwerte als für den Gesundheitszustand des.Patienten kritisch eingestuft wird, oder wobei ein Alarmsignal zweiter Art ausgelöst wird, wenn der erwartete Messwert des Patienten ausbleibt. Die Erfindung ermöglicht es also, von einem Patienten stammende Messwerte zentral abzuspeichern, automatisiert auszuwerten und bei einer Verschlechterung des Gesundheitszustandes eines Patienten oder eines akuten Gesundheitsrisikos eines Patienten ein Alarmsignal erster Art auszulösen, welches gemäß einer Variante der Erfindung automatisch einem den Patienten behandelnden Arzt oder einer medizinische Einrichtung übermittelt wird, um unverzüglich angemessene Maßnahmen zur Behandlung des Patienten einleiten zu können. Auf diese Weise ist ohne großen Verwaltungsaufwand gewährleistet, dass der Patient in relativ kurzer Zeit eine seinem Gesundheitszustand entsprechende Behandlung erhalten kann. Die Auslösung eines Alarmsignals zweiter Art bei Ausbleiben von Messwerten des Patienten ermöglicht es auf vorteilhafte Weise, ohne großen Verwaltungsaufwand, den Patienten zu einer Erfassung der Messwerte anzuhalten, was ebenfalls in einer verbesserten Überwachung des Patienten resultiert. Das bei Ausbleiben von Messwerten ausgelöste Alarmsignal zweiter Art kann dabei direkt an den Patienten und/oder an einen Arzt übermittelt werden, um den Arzt einerseits zu unterrichten, dass Messwerte nicht eingetroffen sind, und andererseits die Möglichkeit einzuräumen, auf den Patienten zur Erfassung der Messwerte einzuwirken. Das Verfahren bzw. das medizinische System bieten dabei außerdem den Vorteil, in derselben Weise den Gesundheitszustand einer Vielzahl von an Epilepsie leidenden Patienten überwachen zu können.

Eine Variante der Erfindung sieht vor, dass wenigstens ein Kriterium für die Auslösung des Alarmsignals erster Art vorzugsweise von einem zu behandelnden Arzt vorgebbar ist. Dabei können Kriterien für die Auslösung des Alarmsignals festgelegt werden, welche für nur einen bestimmten Patienten oder auch für mehrere Patienten gelten. Derartige Kriterien können beispielsweise in vom Arzt festgelegten Toleranzbereichen der Messwerte oder in Sollwerten liegen. Insbesondere ist es nach einer weiteren Variante der Erfindung vorgesehen, dass ein lernendes Expertensystem die Auswertung der Messwerte vornimmt und die Auslösung des Alarmsignals erster Art veranlassen kann.

Eine besonders bevorzugte Ausführungsform der Erfindung sieht vor, dass die Messwerte regelmäßig erfasst werden, wobei, wie bereits erwähnt, ein Alarmsignal zweiter Art immer dann ausgelöst wird, wenn erwartete Messwerte des Patienten ausbleiben.

Nach einer weiteren Ausführungsform der Erfindung wird ein Alarmsignal dritter Art ausgelöst, wenn innerhalb eines vorzugsweise vorgebbaren Zeitfensters keine Reaktion auf das Alarmsignal erster oder zweiter Art erfolgt. Während das Alarmsignal dritter Art bei Ausbleiben einer Reaktion auf das Alarmsignal erster Art an einen Vertreter des Arztes bzw. der medizinischen Einrichtung übermittelt wird, erfolgt die Übermittlung des Alarmsignals dritter Art bei Ausbleiben von Messwerten gegebenenfalls nochmals an den den Patienten behandelnden Arzt.

Varianten der Erfindung sehen vor, dass der oder die Messwerte mit Hilfe eines Fragebogens erfasst werden, wobei der Fragebogen Fragen zur Anamnese, zum Allgemeinzustand, zum Anfallstyp, zur Anfallshäufigkeit, zum Anfallszeitpunkt, zu Komplikationen, zu einem einen Anfall auslösenden Ereignis, zu Medikamenten, zur Dosierung der Medikamente oder zu Medikamentennebenwirkungen aufweist. Der Fragebogen muss dabei nicht notwendigerweise all diese Fragen umfassen. Der Fragebogen kann also individuell an den Patienten angepasst sein.

Eine Ausführungsform der Erfindung sieht vor, dass der oder die Messwerte vom Patienten eigenverantwortlich, von einem Pflegedienst oder von einem Call Center erfasst und der Datenbank zur Speicherung über ein Kommunikationsnetz übermittelt werden. Die Datenbank ist dabei vorzugsweise über ein öffentliches Kommunikationsnetz erreichbar. Auf diese Weise kann sowohl der Patient, als auch das Call Center, als auch ein Pflegedienst, beispielsweise per E-Mail oder durch eine entsprechende Zugangsberechtigung zur Datenbank unter Angabe einer Patientenidentifikationsnummer, die Messwerte einer in der Datenbank geführten Patientenakte hinzufügen. Eine andere Variante der Erfindung sieht vor, dass die in der Datenbank gespeicherten und/oder die ausgewerteten Messwerte über ein Kommunikationsnetz, vorzugsweise über das Internet, abfragbar sind. Demnach kann jede über eine entsprechende Berechtigung verfügende Person, also beispielsweise ein Arzt oder auch ein von einem Arzt beauftragtes Call Center, Messwerte aus der Datenbank abfragen, so dass der Arzt die Messwerte weiter auswerten oder anhand der Messwerte eine Diagnose erstellen und eine Therapie festlegen kann. Eine weitere Ausführungsform der Erfindung sieht dabei vor, dass die Diagnose und/oder die Therapie eines Patienten betreffende Eingaben in eine Patientenakte der Datenbank über das Kommunikationsnetz vornehmbar sind. Die in der Datenbank vorhandene Patientenakte enthält demnach nicht nur die regelmäßig erfassten und ausgewerteten Messwerte, sondern auch die vom Arzt vorgenommenen Diagnosen und eingeleiteten Therapiemaßnahmen.

Ein Ausführungsbeispiel der Erfindung ist in den beigefügten schematischen Zeichnungen dargestellt. Es zeigen:
- Fig. 1: ein medizinisches System zur Überwachung eines an Epilepsie leidenden Patienten in einer außerklinischen Umgebung,
- Fig. 2: eine Benutzeroberfläche zur Konfiguration eines zentralen Alarmgebers, und
- Fig. 3: eine Benutzeroberfläche zur Konfiguration der Weiterleitung von Alarmen.

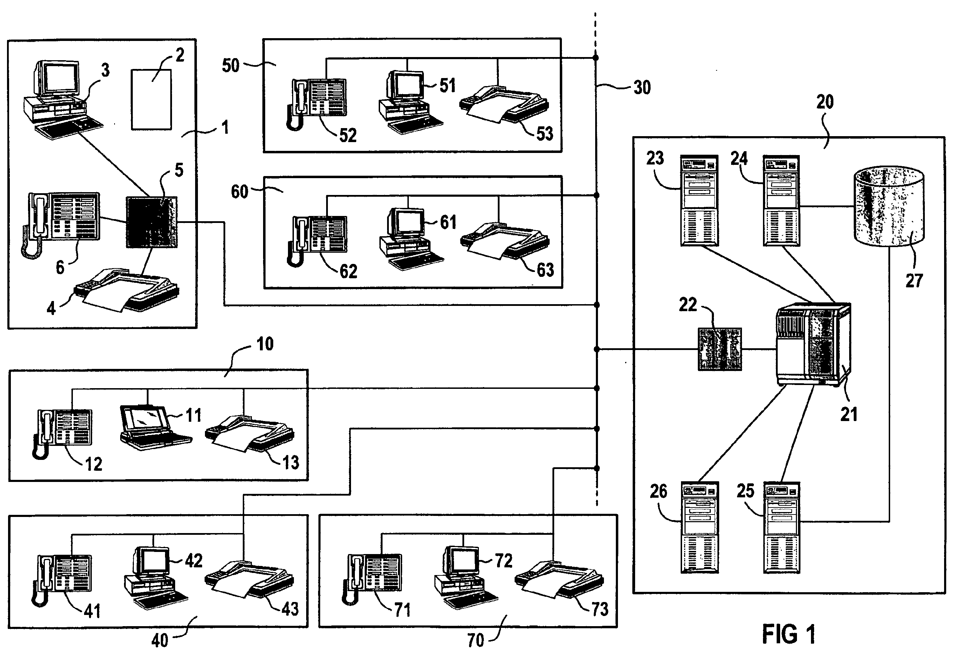

In der Fig. 1 ist ein System zur Überwachung wenigstens eines an Epilepsie leidenden Patienten außerhalb einer medizinischen Einrichtung dargestellt. Zur Überwachung des Patienten werden das Epilepsieleiden des Patienten betreffende Messwerte erfasst, was im Falle des vorliegenden Ausführungsbeispiels im Haus 1 des nicht explizit dargestellten Patienten mit Hilfe eines Fragebogens erfolgt. Der in Fig. 1 schematisch dargestellte Fragebogen 2 weist Fragen zur Anamnese, zum Allgemeinzustand, zum Anfallstyp, zur Anfallshäufigkeit, zum Anfallszeitpunkt, zu Komplikationen, zu einem einen Anfall auslösenden Ereignis, zu Medikamenten, zur Dosierung der Medikamente oder zu Medikamentennebenwirkungen auf. Der Fragebogen kann dabei auch individuell auf einen Patienten abgestimmt sein und andere, weniger oder auch mehr Fragen zur Charakterisierung seines Zustandes aufweisen. Der Fragebogen muss dabei nicht notwendigerweise in Papierform, sondern kann auch als Datei auf einem Rechner 3 vorliegen.

Die Erfassung der Messwerte, also das Ausfüllen des Fragebogens sei es in Papierform 2 oder sei es am Rechner 3, kann eigenverantwortlich durch den Patienten oder durch eine den Patienten besuchende Pflegeperson eines Pflegedienstes 10 erfolgen, welche einen eigens von dem Pflegedienst 10 zur Verfügung gestellten Rechner, beispielsweise in Form eines Laptops 11, zum Ausfüllen des Fragebogens mitführen kann.

Das vorzugsweise zu regelmäßigen Zeitpunkten mit dem Fragebogen zu erfassende Bündel von Messwerten kann per Rechner 3, per Faxgerät 4 oder per Laptop 11 des Pflegedienstes 10 an eine Systemzentrale 20 übermittelt werden. Dies erfolgt über ein Kommunikationsnetz, beispielsweise über ein ISDN-Netz 30, an das beispielsweise der Rechner 3 und das Faxgerät 4 des Patienten über ein ISDN-Interface 5 angeschlossen sind. Der Laptop 11 des Pflegedienstes 10 kann ebenfalls an das Kommunikationsnetz 30 angeschlossen werden. Die vom Pflegedienst erfassten Messdaten können aber auch per Telefon 12 oder per Faxgerät 13 über das Kommunikationsnetz 30 an die Systemzentrale 20 übermittelt werden.

Eine weitere Möglichkeit die Messwerte von dem Patienten zu erfassen liegt in der Inanspruchnahme der Dienstleistung eines Call Centers 40. Ein Angestellter des Call Centers 40 erfasst dabei zu regelmäßigen Zeitpunkten per Telefon 6 bzw. 41 die Messdaten des Patienten und übermittelt diese anschließend ebenfalls per Telefon 41, per Rechner 42 oder per Faxgerät 43 über das Kommunikationsnetz 30 an die Systemzentrale 20.

Bevorzugt erfolgt die Übermittlung der Messwerte per Rechner in Dateiform unter Angabe einer Patientenidentifikation.

Die Systemzentrale 20 dient zur Entgegennahme und Speicherung der Messwerte. Weiterhin soll sie eine Weiterverarbeitung der Messwerte und gegebenenfalls eine Auslösung von Alarmsignalen bewirken.

In der Systemzentrale 20 ist hierzu ein Gateway 21 vorgesehen, das über ein ISDN-Interface 22 an dem ISDN-Netz 30 angeschlossen ist. An dem Gateway 21 kann ein Internet-Proxy-Server 23 zum Zugriff auf das Internet, eine Auswertevorrichtung 24 für die Messwerte, ein Patientendaten-Server 25 zur Verwaltung der Patientendaten und ein Kommunikations-Server 26 als Routingvorrichtung zur Zusammenarbeit aller Komponenten und Weiterleitung von Informationen angeschlossen sein. Mit der Auswertevorrichtung 24 und mit dem Patientendaten-Server 25 ist eine Datenbank 27 als Speichervorrichtung für die Messwerte verbunden.

In der Systemzentrale 20 werden die Messwerte über das ISDN-Interface 22 angenommen und in der Datenbank 27 langfristig abgespeichert. Die Messwerte werden dabei abhängig von der Art der Übermittlung und soweit noch nicht vorhanden in eine maschinenlesbare Datei umgesetzt. Die Auswertevorrichtung 24, bei der es sich um einen handelüblichen Rechner handeln kann, wird mit einer Auswertesoftware betrieben, welche einerseits die Messwerte mit vorgegebenen, in dem Datenspeicher 27 gespeicherten Kriterien für die Überwachung von an Epilepsie leidenden Patienten vergleicht und andererseits den Zeitpunkt der Übertragung der Messwerte auswertet. Die Kriterien für die Überwachung eines oder mehrerer an Epilepsie leidender Patienten sowie die Zeitpunkte, zu denen Messwerte für die Überwachung von dem oder den Patienten vorhanden sein müssen, sind dabei vorgebbar. Je nachdem wo sich der oder die Patienten in Behandlung befinden, können die Kriterien sowie die Messzeitpunkte von einem freiberuflichen Arzt, dessen Praxis in Fig. 1 mit 50 bezeichnet ist, oder einem Arzt einer medizinischen Einrichtung, beispielsweise einer Klinik 60, vorgegeben werden. Die Kommunikation des jeweiligen Arztes mit der Systemzentrale 20, in deren Datenbank 27 die entsprechenden Vorgaben patientenspezifisch für die Auswertevorrichtung 24 verfügbar gehalten werden, kann dabei auf direktem Wege mittels eines Rechners 51 bzw. 61, einer Fernsprecheinrichtung 52 bzw. 62 oder eines Faxgerätes 53 bzw. 63 erfolgen. Der oder die Ärzte können sich für die Übermittlung der Vorgaben aber auch der Dienstleistung des Call Centers 40 bedienen, welches die Vorgaben des oder der Ärzte telefonisch entgegen nimmt und an die Systemzentrale 20 übermittelt.

Kriterien für die Überwachung eines an Epilepsie leidenden Patienten stellen beispielsweise die Vorgabe von Toleranzgrenzen für die Häufigkeit von epileptischen Anfällen oder Sollwerte für die Einnahme von Medikamenten dar.

Eine berechtigter Arzt kann schließlich die ausgewerteten Messwerte von der Datenbank 27 direkt abfragen bzw. beispielsweise per Call Center 40 abfragen lassen und Diagnosen und Therapien erstellen. Der Arzt kann hierzu Eintragungen in einer in der Datenbank 27 gespeicherten Patientenakte vornehmen, in der auch die originalen und ausgewerteten Messwerte gespeichert sind.

Die Auswertevorrichtung 24 umfasst außerdem einen Alarmgeber zur Erzeugung von Nachrichten und zur Auslösung von Alarmsignalen. In der Systemzentrale 20 werden dabei alle Informationen zusammengeführt, die zur Auslösung von Alarmsignalen und gegebenenfalls zur Weiterleitung von Alarmsignalen und Nachrichten bei Abwesenheit des vorgesehenen Empfängers benötigt werden. Neben der Arztpraxis 50, der Klinik 60, dem Patienten, dem Pflegedienst 10 und dem Call Center 40 ist mit der Systemzentrale 20 daher auch eine Praxis 70 einer Ärzte-Bereitschaft über ein Telefon 71, einen Personal Computer 72, ein Faxgerät 73 oder ein nicht dargestelltes Handy verbunden, an die ein Alarmsignal weitergeleitet werden kann, um Maßnahmen zur Behandlung eines betroffenen Patienten einzuleiten.

Ein Alarmsignal erster Art wird von dem Alarmgeber beispielsweise bei signifikanten Unter- bzw. Überschreitung der Messwerte von Toleranz- bzw. Sollwertgrenzen ausgelöst. Das Alarmsignal erster Art wird beispielsweise an den erstbehandelnden, freiberuflichen Arzt oder an den erstbehandelnden Arzt der Klinik 60 über das Kommunikationsnetz 30 übermittelt. Der jeweils behandelnde Arzt, welcher über eine entsprechende Berechtigung verfügt, kann dabei die in der Datenbank 27 gespeicherten und ausgewerteten Messwerte mit dem Rechner 51 bzw. 61, dem Telefon 52 bzw. 62, dem Faxgerät 53 bzw. 63, einem Handy oder einer anderen Kommunikationseinrichtung abrufen, um basierend auf den Messwerten geeignete Maßnahmen zur Behandlung des Patienten einzuleiten.

Zur Auswertung der Messwerte und zur Auslösung von Alarmsignalen kann ein Expertensystem eingesetzt werden, das Teil der Auswertevorrichtung 24 ist und die Messwerte problemspezifisch anhand von Regelsystemen oder auf Wahrscheinlichkeiten basierend interpretiert. Dabei ist eine Individualisierung des Expertensystems auf den Patienten vorgesehen, d.h. das Expertensystem lernt den jeweiligen an Epilepsie leidenden Patienten im Überwachungsprozess immer besser kennen, es trifft dazu als lernendes System ständig Prognosen über zu erwartende zukünftige Messwerte, die es mit den wahren Messwerten vergleicht. Damit wird eine individualisierte Überwachung realisiert.

Ein Alarmsignal zweiter Art wird von dem Alarmgeber immer dann ausgelöst, wenn fällig gewordene Messwerte vom Patienten nicht geliefert worden sind. Das Alarmsignal zweiter Art kann dabei dem Patienten als Auforderung zur Übersendung der Messwerte als auch dem jeweils behandelnden Arzt zur Kenntnisnahme der verzögerten Erfassung der Messwerte bzw. zur Einwirkung auf den Patienten, die Messwerte zu erfassen und der Datenbank 27 zu übertragen, übermittelt werden.

Ein Alarmsignal dritter Art wird immer dann ausgelöst, wenn eine Reaktion eines behandelnden Arztes innerhalb eines vorzugsweise vorgebbaren und von der Dringlichkeit des Falles abhängigen Zeitfensters auf ein Alarmsignal erster Art hin ausbleibt. Des weitere wird ein Alarmsignal dritter Art immer dann ausgelöst, wenn der Patient oder der Arzt auf ein Alarmsignal zweiter Art nicht innerhalb des Zeitfensters reagiert. Das Alarmsignal dritter Art wird vorzugsweise an die Ärztebereitschaft 70 übermittelt, um geeignete Maßnahmen sei es zur Anleitung oder sei es zur Behandlung des Patienten einzuleiten.

Die Alarmsignale können in Abhängigkeit von der Dringlichkeit einer Reaktion einer benachrichtigten Person oder Institution in unterschiedlichen Dringlichkeitsstufen generiert werden, beispielsweise dringendst, dringend, Routine oder Standard. Welche Messwerte zu welchen Dringlichkeitsstufen führen, kann mit den gleichen Mechanismen wie für die Auswertung der Messwerte festgelegt werden und in der Datenbank 27 abgespeichert sein. Beispielsweise können vier verschiedene Toleranzbereiche für die Messwerte festgelegt werden, wobei im ersten Toleranzbereich liegende Messwerte ein Alarmsignal erster Art der Stufe Standard und im vierten Toleranzbereich liegende Messwerte ein Alarmsignal erster Art der Stufe dringendst auslösen.

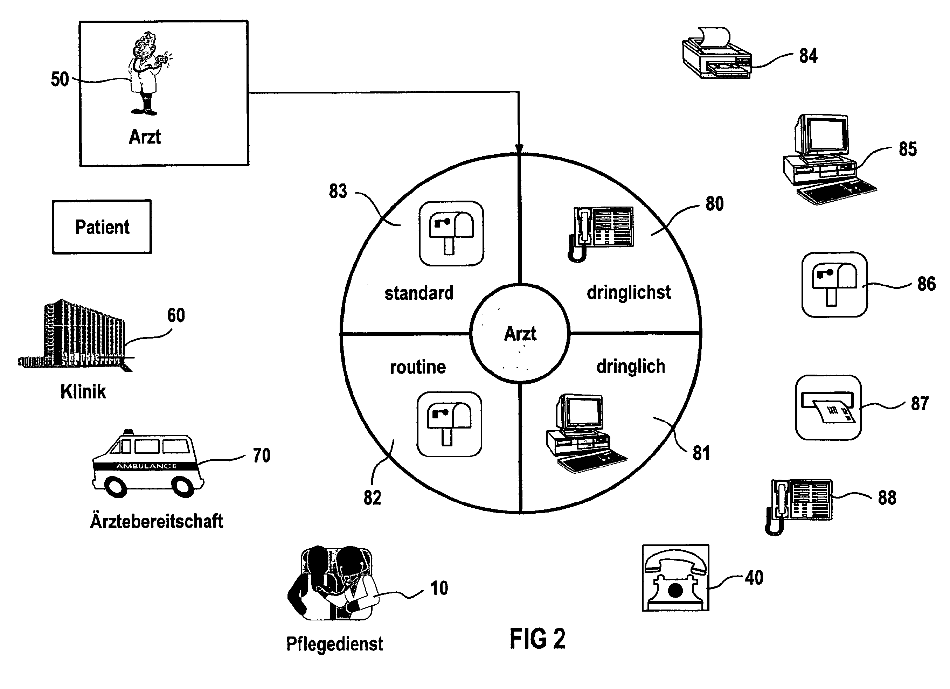

Die Konfiguration des zentralen Alarmgebers erfolgt individuell durch den jeweiligen Anwender. So legt z.B. der freiberufliche Arztes für sich selbst fest, wie er Nachrichten unterschiedlicher Dringlichkeit erhalten will. Dazu verwendet er die in Fig. 2 gezeigte Benutzeroberfläche (user interface). Der Kommunikationskreis ist in Segmente 80 bis 83 unterteilt, die die Dringlichkeitsstufen der Alarmnachrichten repräsentieren.

Zur Konfiguration zieht der Arzt jeweils die ihm zur Verfügung stehenden Informationswege bzw. die an dem Kommunikationsnetz 30 angeschlossenen Kommunikationsgeräte aus der Reihe der angebotenen Informationswege und Kommunikationsgeräte 85 bis 88 in die entsprechenden Segmente 80 bis 83, über die er bei Eintreffen einer klassifizierten Nachricht erreichbar sein möchte. Die Konfiguration wird in der Systemzentrale 20 gespeichert.

Bei der gewählten Konfiguration gemäß Fig. 2 wird der Arzt bei dringlichsten Fällen per Telefon, bei dringlichen Fällen per E-Mail über einen Rechner und in allen anderen Fällen per Post benachrichtigt.

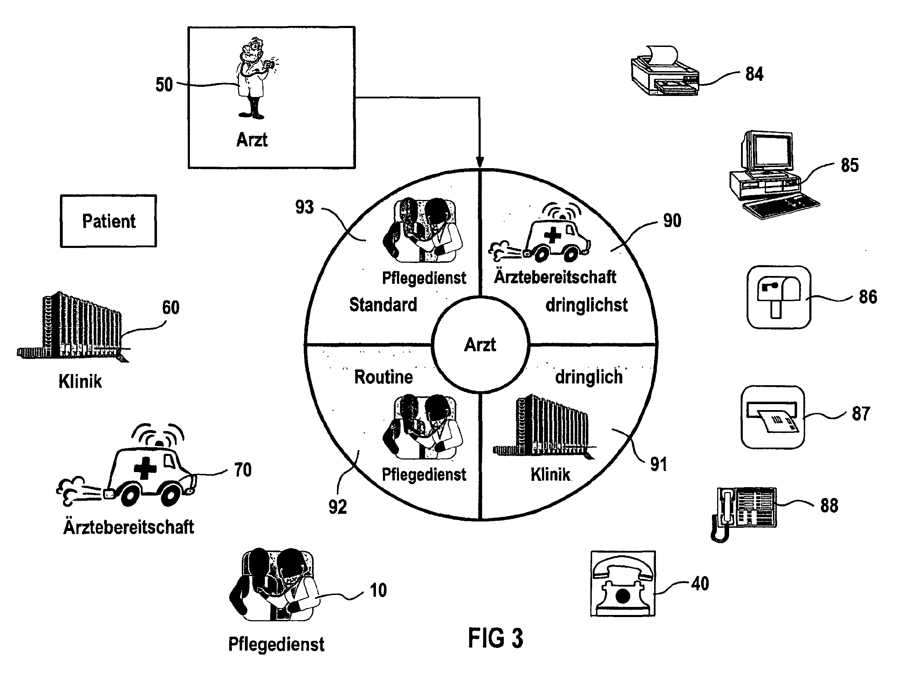

Wenn ein Arzt auf ein Alarmsignal erster bzw. zweiter Art nicht in angemessener Zeit, d. h. innerhalb des Zeitfensters, reagiert, löst der Alarmgeber der Auswertevorrichtung 24 in der Systemzentrale 20 ein Alarmsignal dritter Art aus. Die Konfiguration, an wen das Alarmsignal dritter Art übermittelt werden sollen, trifft der Arzt selbst. Er verwendet hierzu einen in Fig. 3 dargestellten Kommunikationskreis, der anhand der Dringlichkeitsstufen segmentiert ist. Er zieht in die entsprechenden Kreissegmente die Institutionen 10, 40, 60, 70 hinein, die im Notfall zu alarmieren sind, wenn er selbst nicht reagiert.

Bei der gewählten Konfiguration gemäß Fig. 3 wird bei dringlichsten Fällen die Ärztebereitschaft, bei dringlichen Fällen die Klinik und in allen anderen Fällen der Pflegedienst benachrichtigt.

Es kann vorgesehen sein, dass die Festlegung der Art der Alarmierung des Arztes und die Vorgabe des Empfängers des Alarmsignals dritter Art in einem einzigen Kommunikationskreis erfolgen.

Es ist zusätzlich vorgesehen, dass Default-Einstellungen vom Server vorgegeben werden, so dass beim Neueinrichten dem Arzt ein sinnvoller Vorschlag vorgegeben wird, wobei eine Präferenzliste des Arztes berücksichtigt wird.

Auch der Informationskanal zum Patienten, zum Pflegedienst, zur Klinik oder zur Ärztebereitschaft lässt sich konfigurieren, so dass festgelegt werden kann, in welcher Weise die Institutionen im Falle der verschiedenen Arten von Alarmsignalen und Alarmstufen kontaktiert werden wollen. Dabei wird jeweils ein dem in Fig. 2 gezeigten Kommunikationskreis entsprechender Kommunikationskreis konfiguriert, in den die Erreichbarkeitswege eintragen werden.

Ebenso ist vorgesehen, dass der Patient eine Weiterleitung dringender Nachrichten im Urlaub oder bei Krankenhausaufenthalt einstellen kann.

Die Ärzte, Patienten, Pflegedienste und Ärztebereitschaften können sich als Übermittlungsweg von Alarmsignalen oder anderweitigen Informationen auch jeweils des Call Centers 40 bedienen, wobei dem Call Center 40 Informationen vorliegen müssen, an wen beispielsweise ein Alarmsignal dritter Art weitergeleitet werden soll.

Das Versenden von Informationen, z. B. von Alarmsignalen, geht dabei derart vonstatten, dass die Auswertevorrichtung 24 ein Alarmsignal erzeugt und dieses an den Kommunikations-Server 26 zum Versand weitergibt. Der Kommunikations-Server 26 als Routingvorrichtung versendet das Alarmsignal an einen Empfänger unter Zuhilfenahme einer Unterkomponente Telefonbuch, in dem die Kontaktinformationen zu einem adressierbaren Empfänger bereitgestellt werden.

Bei den von Patienten, Ärzten, Pflegediensten und/oder Ärztebereitschaften verwendeten Endgeräte kann es sich um PDAs (Personal Digital Assistent) mit Modem, Telefone mit Sprache oder Tonwahl (DTMF), TV Set-Top-Boxes, Handys, internetfähige Rechner mit WWW-Formularen, Faxgeräte oder andere Gerät zur Informationsübertragung handeln. Informationen wie Alarmsignale können demnach über Sprachausgabe per Telefon, per SMS, per E-Mail, per WWW, per WAP, per Fax, per proprietären Dienst oder per Briefpost übermittelt werden.

Die Endgeräte der Patienten, Ärzte, Pflegedienste und/oder Ärztebereitschaften weisen vorzugsweise eine auf Internet-Technologien basierende grafische Benutzeroberfläche auf. Zur Parametrierung des Alarmgebers bieten die Endgeräte das in Fig. 2 visualisierte Verfahren an. Zur Parametrierung der Benachrichtigungswege dient das anhand Fig. 3 dargestellte Verfahren.

Spezielle Ausführungen des Alarmgebers ermöglichen eine Alarmauslösung bei fehlerhaft erfassten Messwerten und eine Alarmauslösung bei Compliance-Problemen des Patienten.

Die Auswertevorrichtung 24 für die Messwerte und der Patientendaten-Server 25 können zur Datenerhebung so ausgeführt sein, dass die Messwerte sicher und in pseudonymer Form gespeichert werden.

Durch das erfindungsgemäße Verfahren und System wird also eine Möglichkeit zur Überwachung eines an Epilepsie leidenden Patienten außerhalb einer medizinischen Einrichtung geschaffen, die ein zentrales Alarmgebersystem aufweist, das Alarmsignale generiert bei:
- Überschreiten von vorgegebenen Grenzwerten (d.h. die Messwerte liegen in einem abnormen Bereich)
- Mangelhafter Patientencompliance (erwartete Messwerte des Patienten bleiben aus oder der Patient reagiert nicht auf ein Alarmsignal)
- Mangelhafter Arztcompliance (erwartete Reaktion auf ein Alarmsignal durch den Arzt bleibt aus).

Ein Konfigurationskonzept für die Erreichbarkeit und die Alarmweiterleitung ermöglicht die Einstellungen nach den aktuellen Bedürfnissen jedes Betreuungsfalles.
- Eskalatationsmechanismen sorgen bei fehlender Reaktion eines Arztes für eine Weiterleitung.
- Eine Konfiguration der Datenkommunikation vom Arzt und/oder Pfleger zum Patienten kann durch den Patienten erreicht werden.

## Patentansprüche

1. Verfahren zur Überwachung wenigstens eines an Epilepsie leidenden Patienten außerhalb einer medizinischen Einrichtung, aufweisend folgende Verfahrensschritte:
a) Erfassung wenigstens eines das Epilepsieleiden betreffenden Messwertes des Patienten,
b) Übermittlung und Speicherung des Messwertes in einer zentralen Datenbank (27),
c) Auswertung des Messwertes und
d) Auslösung eines Alarmsignals erster Art, wenn der ausgewertete Messwert als für den Gesundheitszustand des Patienten kritisch eingestuft wird, oder Auslösung eines Alarmsignals zweiter Art, wenn der Messwert des Patienten ausbleibt.

2. Verfahren nach Anspruch 1, bei dem das Alarmsignal erster Art automatisch einem den Patienten behandelnden Arzt (50) oder einer medizinischen Einrichtung (60) übermittelt wird.

3. Verfahren nach Anspruch 1 oder 2, bei dem wenigstens ein Kriterium für die Auslösung des Alarmsignals erster Art festlegbar ist.

4. Verfahren nach einem der Ansprüche 1 bis 3, bei dem die Auswertung des Messwertes mit Hilfe eines lernenden Expertensystems erfolgt, welches die Auslösung des Alarmsignals erster Art veranlassen kann.

5. Verfahren nach einem der Ansprüche 1 bis 4, bei dem der Messwert regelmäßig erfasst wird.

6. Verfahren nach einem der Ansprüche 1 bis 5, bei dem ein Alarmsignal dritter Art ausgelöst wird, wenn innerhalb eines Zeitfensters keine Reaktion auf das Alarmsignal erster oder zweiter Art erfolgt.

7. Verfahren nach einem der Ansprüche 1 bis 6, bei dem der Messwert mit Hilfe eines Fragebogens (2) erfasst wird.

8. Verfahren nach Anspruch 7, bei dem der Fragebogen (2) Fragen zur Anamnese, zum Allgemeinzustand, zum Anfallstyp, zur Anfallshäufigkeit, zum Anfallszeitpunkt, zu Komplikationen, zu einem einen Anfall auslösenden Ereignis, zu Medikamenten, zur Dosierung der Medikamente oder zu Medikamentennebenwirkungen aufweist.

9. Verfahren nach einem der Ansprüche 1 bis 8, bei dem der Messwert vom Patienten eigenverantwortlich, von einem Pflegedienst (10) oder von einem Call Center (40) erfasst und der Datenbank (27) zur Speicherung über ein Kommunikationsnetz (30) übermittelt wird.

10. Verfahren nach einem der Ansprüche 1 bis 9, bei dem der in der Datenbank (27) gespeicherte und/oder der ausgewertete Messwert über ein Kommunikationsnetz (30) abfragbar ist.

11. Verfahren nach einem der Ansprüche 1 bis 10, bei dem die Diagnose und/oder die Therapie eines Patienten betreffende Eingaben in eine Patientenakte der Datenbank (27) über ein Kommunikationsnetz (30) vornehmbar sind.

12. Medizinisches System zur Überwachung wenigstens eines an Epilepsie leidenden Patienten außerhalb einer medizinischen Einrichtung aufweisend eine Datenbank (27) für die zentrale Speicherung wenigstens eines von dem Patienten stammenden, das Epilepsieleiden betreffenden Messwertes, eine Auswertevorrichtung (24) für den Messwert und einen Alarmgeber (24), welcher ein Alarmsignal erster Art auslöst, wenn der ausgewertete Messwert als für den Gesundheitszustand des Patienten kritisch eingestuft wird, oder welcher ein Alarmsignal zweiter Art ausgelöst, wenn der Messwert des Patienten ausbleibt.

13. Medizinisches System nach Anspruch 12, bei dem das Alarmsignal erster Art automatisch einem den Patienten behandelnden Arzt (50) oder einer medizinischen Einrichtung (60) übermittelt wird.

14. Medizinisches System nach Anspruch 12 oder 13, bei dem wenigstens ein Kriterium für die Auslösung des Alarmsignals erster Art festlegbar ist.

15. Medizinisches System nach einem der Ansprüche 12 bis 14, bei dem die Auswertung des Messwertes mit Hilfe eines lernenden Expertensystems erfolgt, welches die Auslösung des Alarmsignals erster Art veranlassen kann.

16. Medizinisches System nach einem der Ansprüche 12 bis 15, bei dem der Messwert regelmäßig erfasst wird.

17. Medizinisches System nach einem der Ansprüche 12 bis 16, bei dem ein Alarmsignal dritter Art ausgelöst wird, wenn innerhalb eines Zeitfensters keine Reaktion auf das Alarmsignal erster oder zweiter Art erfolgt.

18. Medizinisches System nach einem der Ansprüche 12 bis 17, bei dem der Messwert mit Hilfe eines Fragebogens (2) erfasst wird.

19. Medizinisches System nach Anspruch 18, bei dem der Fragebogen (2) Fragen zur Anamnese, zum Allgemeinzustand, zum Anfallstyp, zur Anfallshäufigkeit, zum Anfallszeitpunkt, zu Komplikationen, zu einem einen Anfall auslösenden Ereignis, zu Medikamenten, zur Dosierung der Medikamente oder zu Medikamentennebenwirkungen aufweist.

20. Medizinisches System nach einem der Ansprüche 12 bis 19, bei dem der Messwert vom Patienten eigenverantwortlich, von einem Pflegedienst (10) oder von einem Call Center (40) erfasst und der Datenbank (27) zur Speicherung über ein Kommunikationsnetz (30) übermittelt wird.

21. Medizinisches System nach einem der Ansprüche 12 bis 20, bei dem der in der Datenbank (27) gespeicherte und/oder der ausgewertete Messwert über ein Kommunikationsnetz (30) abfragbar ist.

22. Medizinisches System nach einem der Ansprüche 12 bis 21, bei dem die Diagnose und/oder die Therapie eines Patienten betreffende Eingaben in eine Patientenakte der Datenbank (27) über ein Kommunikationsnetz (30) vornehmbar sind.
